# EUROPÄISCHE PATENTSCHRIFT

(11) **EP 1 779 712 B1**
(45) Veröffentlichungstag und Bekanntmachung des Hinweises auf die Patenterteilung: **20.04.2011**
(21) Anmeldenummer: 05756502.0
(22) Anmeldetag: 16.06.2005
(51) Int. Cl.: H05H 13/04

(54) **TEILCHENBESCHLEUNIGER FÜR DIE STRAHLENTHERAPIE MIT IONENSTRAHLEN**
PARTICLE ACCELERATOR FOR RADIOTHERAPY BY MEANS OF ION BEAMS
ACCELERATEUR DE PARTICULES POUR LA RADIOTHERAPIE PAR FAISCEAUX IONIQUES

(30) Priorität: 16.06.2004 DE 202004009421 U
(43) Veröffentlichungstag der Anmeldung: 02.05.2007
(73) Patentinhaber: GSI Helmholtzzentrum für Schwerionenforschung GmbH, 64291 Darmstadt (DE)
(72) Erfinder: BLASCHE, Klaus, 64291 Darmstadt (DE); FRANCZAK, Bernhard, 64291 Darmstadt (DE)
(74) Vertreter: Forstmeyer, Dietmar
(86) Internationale Anmeldenummer: PCT/EP2005/006491
(87) Internationale Veröffentlichungsnummer: WO 2005/125289

(56) Entgegenhaltungen:
- EP-A- 0 994 638
- US-A- 4 992 746
- US-A- 5 285 166
- US-A- 6 008 499
- US-A- 6 087 670
- US-A1- 2002 014 588
- HATTORI T ET AL: "A study of a test APF-IH type linac as an injector for cancer therapy" NUCLEAR INSTRUMENTS & METHODS IN PHYSICS RESEARCH, SECTION - B: BEAM INTERACTIONS WITH MATERIALS AND ATOMS, NORTH-HOLLAND PUBLISHING COMPANY. AMSTERDAM, NL, Bd. 188, Nr. 1-4, April 2002 (2002-04), Seiten 221-224, XP004346478 ISSN: 0168-583X

## Beschreibung

Die Erfindung betrifft einen Teilchenbeschleuniger für die Strahlentherapie mit Ionenstrahlen. Ein derartiger Teilchenbeschleuniger ist aus der Druckschrift DE 100 10 523 C2 bekannt und weist als Komponenten des Teilchenbeschleunigers unterschiedliche Ionenquellen auf, die unterschiedliche Materialien ionisieren, ein Massenspektrometer zum Selektieren der Ionen, einen Beschleuniger zur linearen Vorbeschleunigung der Ionen, Injektionsmittel zum Einleiten der Ionen in einen sechszähligen Synchrotronring. Der sechszählige Synchrotronring dient zur weiteren Hochbeschleunigung der Ionen. Ferner weist der bekannte Teilchenbeschleuniger Extraktionsmittel zum Auskoppeln der hochbeschleunigten Ionen aus dem Synchrotronring in eine Strahlzuführungsstrecke mit Umlenkmagneten zu entsprechenden Patientenbestrahlungsplätzen auf.

Kernstück der Hochbeschleunigung eines Ionenstrahls in einem Teilchenbeschleuniger für die Strahlentherapie mit Ionenstrahlen ist der Synchrotronring, in dem den Ionen durch mehrere gesteuerte Umläufe in dem Synchrotronring nach einem Beschleunigungszyklus genau soviel Energie zugeführt wird, um ein Volumenelement eines kranken Gewebes in einer vorgegebenen Tiefe zu zerstören, ohne das darüber liegende gesunde Deckgewebe zu verletzen. In einem Synchrotron werden demnach von Zyklus zu Zyklus unterschiedliche Strahldosierungen mit jeweils nur soviel Strahlungsenergie erzeugt, wie es für das Erreichen unterschiedlicher vorgegebener Tiefen erforderlich ist, im Gegensatz zu einem Zyklotron. In dem Zyklotron wird eine maximal vorgegebene Strahlenenergie gleich bleibend von Zyklus zu Zyklus ohne Berücksichtigung des erforderlichen Bedarfs erzeugt, was die Strahlenbelastung der Umwelt erhöht, da die jeweils erforderliche Strahlenenergie nach der Beschleunigung durch Abbremsen in Absorbern geeigneter Dicke eingestellt wird. Bei diesem Prozess kann nur ein kleiner Teil des beschleunigten Teilchenstrahls für die Therapiebestrahlung genutzt werden, was die Umweltschutz-Richtlinien verletzt.

Der aus obiger Druckschrift bekannte sechszählige Synchrotronring mit sechs geradlinigen Strahlstrecken und sechs gebogenen Strahlstrecken weist auf einer ersten geradlinigen Strahlstrecke Injektionsmittel für das Einführen eines linearbeschleunigten Ionenstrahls in den Synchrotronring auf und besitzt im Verlauf einer zweiten geradlinigen Strahlstrecke mindestens ein Beschleunigungsmittel für den Ionenstrahl und koppelt an einer dritten geradlinigen Strahlstrecke über Extraktionsmittel nach mehreren Umläufen am Ende eines Zyklus den dosiert hochbeschleunigten Ionenstrahl in die Strahlzuführungsstrecke aus. Darüber hinaus sind in dem bekannten Synchrotronring 3 Bumpermagnete auf den geraden Strahlstrecken angeordnet, die nach Injektion der Ionen in den Synchrotronring in mehreren Umläufen den Ionenstrahl zentrieren, wobei einer der Bumpermagnete in der geradlinigen Strahlstrecke angeordnet ist, in der auch Injektionsmittel angeordnet sind.

Dazu sind in dem bekannten sechzähligen Synchrotronring in jeder der gebogenen Strahlstrecken zur horizontalen Umlenkung des Ionenstrahls um 60° ein langgestreckter, massiver und tonnenschwerer Dipolmagnet mit einer Spulen- und Polschuhkonfiguration des H-Typs angeordnet und strahlaufwärt sind vor dem Eintritt des Ionenstrahls in die Apertur des Dipolmagneten zur horizontalen Stabilisierung des Ionenstrahls ein horizontal fokussierender Quadrupolmagnet und ein horizontal defokussierender Quadrupolmagnet hintereinander angeordnet.

Ein Nachteil des bekannten Teilchenbeschleunigers mit Synchrotronring ist der lange Weg, den der Ionenstrahl durch den Dipolmagneten zurücklegen muss, bis die nächste geradlinige Strahlstrecke erreicht ist. Dies erfordert eine sich weit öffnende Apertur. Damit ist nachteilig der Einsatz von einem materialtechnisch aufwendigen Dipolmagneten mit einem großen Bedarf an elektrischer Pulsleistung verbunden, was die Investitionskosten für die Magnet und Magnetstromversorgungen sowie die Betriebskosten heraufsetzt. Hinzukommen erhöhte technische Anforderungen an die Fundamente, auf denen die langgestreckten, massiven und tonnenschweren Dipolmagnete ortstabil anzuordnen sind, was die Bau- und Investitionskosten belastet. Schließlich ergeben sich auch Wartungs- und Instandhaltungsprobleme, da schweres den zu bewegenden Massen angepasstes Hub- und Transportgerät erforderlich ist, was die Betriebskosten heraufsetzt. Darüber hinaus erfordern nachteilig die großen Dimensionen der Dipolmagnete den Einsatz von mindestens zwei Septummagneten als Extraktionsmittel, die in einer geradlinigen Strahlstrecke in der Lage sind, den Strahl beim Extrahieren an den Dipolmagneten vorbei aus dem Synchrotronring herauszuführen.

Für die Strahlentherapie mit Ionenstrahlen hat sich das Konzept der aktiven Raster-Scanning Technik mit einer von Puls zu Puls einstellbaren Energie des Ionenstrahls bewährt. Der optimale Beschleunigertyp für diese Form der Bestrahlungstechnik ist das Synchrotron. In der GSI in Darmstadt wurde das Schwerionen-Synchrotron SIS seit vielen Jahren mit Erfolg für die Entwicklung der Strahlentherapie mit Ionenstrahlen eingesetzt. In der Universitätsklinik in Heidelberg wird zur Zeit eine neue Beschleunigeranlage mit einem kleinen Synchrotron für die klinische Anwendung der Strahlentherapie mit Ionenstrahlen aufgebaut, wie es aus der Druckschrift: The Proposed Dedicated Ion Beam Facility for Cancer Therapy at the Clinic in Heidelberg, EPAC 2000 bekannt ist.

Das Schwerionen-Synchrotron SIS ist eine weit größere Beschleunigeranlage, die nach anderen technischen Konzepten ausgelegt ist. Die Beschleunigeranlage in Heidelberg ist jedoch mit dem oben beschriebenen sechszähligen Synchrotron ausgestattet, das mit den angegebenen Nachteilen in Bezug auf Größe und Schwere der Dipolmagnete und zugeordneter Komponenten wie beispielweise Quadrupole, Bumpermagnete und Septummagnete, sowie in Bezug auf Investitions-, Bau- und Betriebskosten, verbunden ist.

Die US 2002/0014588 A1 beschreibt einen Beschleuniger zum Beschleunigen geladener Teilchen und ein medizinisches System, das dessen Strahl verwendet. Ein Beschleuniger-Ring umfasst vier Dipolpaare. Eine Strahlkonvergenz erzeugende Quadrupolmagnete sind vor einzelnen Dipolmagnetpaaren und eine Strahldivergenz erzeugende Quadrupolmagnete sind zwischen den Dipolmagneten der Dipolmagnetpaare angeordnet.

Die EP 0 994 638 A1 beschreibt ein Verfahren zum Extrahieren eines Strahls geladener Teilchen aus einem Beschleuniger.

Die US 6,008,499 A ist auf die effiziente Ejektion des Strahls aus dem Synchrotron gerichtet. Vorgeschlagen wird eine Anordnung eines oder mehrerer defokussierender Quadrupolmagneten zwischen einem elektrostatischen Deflektor und einem ablenkenden Elektromagneten. Ein Beschleuniger-Ring mit sechs einfachen Dipolmagneten ist gezeigt.

Die US 5,285,166 A beschreibt ein Verfahren zum Extrahieren geladener Teilchen aus einem Beschleuniger. Beschleuniger-Ringe mit sechs gebogenen Abschnitten mit je einem Dipolmagneten sind dargestellt. Vor jedem Dipolmagneten ist ein fokussierende Quadrupolmagnet, nach jedem Dipolmagnet ist ein defokussierender Quadrupolmagnet angeordnet. Bumpermagnete sind an den geraden Strecken vor und nach dem zur Extraktion vorgesehenen Deflektor angeordnet.

Aufgabe der Erfindung ist es, einen Teilchenbeschleuniger für die Strahlentherapie mit Ionenstrahlen zu schaffen, der die Nachteile im Stand der Technik überwindet, und einen Teilchenbeschleuniger für die Strahlentherapie mit Ionenstrahlen zur Verfügung stellt, der vollelektronisch steuerbar und zuverlässig einen Präzisionsionenstrahl für die Strahlentherapie mit Ionenstrahlen bereit stellt.

Diese Aufgabe wird mit dem Gegenstand des unabhängigen Anspruchs 1 gelöst. Vorteilhafte Weiterbildungen der Erfindung ergeben sich aus den abhängigen Ansprüchen.

So wird erfindungsgemäß ein Teilchenbeschleuniger für die Strahlentherapie mit Ionenstrahlen vorgesehen, wobei der Teilchenbeschleuniger einen sechszähliegen Synchrotronring mit sechs geradlinigen Strahlstrecken und sechs gebogenen Strahlstrekken aufweist, wobei von den sechs geradlinigen Strahlstrecken:
- auf einer geradlinigen Strahlstrecke Injektionsmittel für das Einführen eines linearbeschleunigten Ionenstrahl in den Synchrotronring angeordnet sind,
- im verlauf einer anderen geradlinigen Strahlstrecke mindestens ein Beschleunigungsmittel für den Ionenstrahl vorhanden ist,
- an einer weiteren geradlinigen Strahlstrecke Extraktionsmittel für das Extrahieren des nach mehreren Umläufen hochbeschleunigten Ionenstrahls vorgesehen sind,
- jede gebogene Strahlstrecke ein Dipolmagnetpaar aufweist,
- zwischen jedem Dipolmagnetpaar ein horizontal defokussierender Quadrupolmagnet angeordnet ist, und
- strahlaufwärts vor jedem Dipolmagnetpaar in den geradlinigen Strahlstrecken ein horizontal fokussierender Quadrupolmagnet vorgesehen ist.

Der erfindungsgemäße Teilchenbeschleuniger hat den Vorteil, dass die lange gebogenen Strahlstrecke, die bisher von einem langgestreckten, massiven und tonnenschweren Dipolmagneten mit einer Spulen- und Polschuhkonfiguration des H-Typs auf ein Dipolmagnetpaar aus zwei Dipolmagneten verteilt ist. Durch die kürzere.Weglänge des Ionenstrahls innerhalb jeweils einem der Dipole des Dipolpaares ist es in vorteilhafterweise möglich, die Apertur deutlich zu verringern und dementsprechend die Dipole mit geringerem Gewicht und einer verbesserten Spulen- und Polschuhkonfiguration auszustatten. Außerdem ist mit der Anordnung des erfindungsgemäßen Dipolpaares die vorteilhafte Möglichkeit gegeben, den horizontal defokussierenden Quadrupolmagnet zwischen dem Dipolpaar zu positionieren, was die Anforderungen an die Apertur und Quadrupolstärke weiter minimiert. Davon sind vorzugsweise die Dipole eines Dipolmagnetpaares derart eng hintereinander in der gebogenen Strahlstrecke angeordnet, dass exakt ein defokussierender Quadrupol zwischen den Dipolmagneten des Dipolmagnetenpaares angeordnet werden kann.

Vorzugsweise weist das Dipolmagnetpaar eine Spulenkonfiguration aus einer Kombination von Window-Frame-Magnet-Typ und H-Magnet-Typ auf, der auch WF/H-Typ genannt werden kann. Dieser vorteilhafte Magnettyp wird aufgrund der verkürzten Bahnlänge und der verkleinerten Apertur möglich und gestattet, Dipolmagnete mit erheblich kleinerem Querschnitt und entsprechend geringerem Gewicht sowie mit stark reduziertem Bedarf an elektrischer Pulsleistung einzusetzen.

Weiterhin ist es in einer bevorzugten Ausführungsform der Erfindung vorgesehen, die Bumpermagnete außerhalb der geradlinigen Strahlstrecke für die Injektionsmittel in den drei anderen der sechs geradlinigen Strahlstrecken derart anzuordnen, dass ein Bumpermagnet strahlabwärts der Injektionsmittel und mindestens ein Bumpermagnet strahlaufwärts der Injektionsmittel angeordnet sind. Diese Ausführungsform hat den Vorteil, dass die geradlinige Strahlstrecke für die Injektionsmittel nicht überfrachtet wird, so dass gleichbleibend sechs kurze geradlinige Strahlstrecken möglich sind, was sich auf den Gesamtumfang des Synchrotronringes günstig auswirkt.

Weiterhin ist es vorgesehen mit lediglich zwei Bumpermagneten das Zentrieren des Ionenstrahls nach der Injektion der Ionen zu ermöglichen und damit die Investitionskosten weiter zu vermindern.

Vorzugsweise weisen die Bumpermagnete für eine Injektion des Ionenstrahls innerhalb einer begrenzten Zahl von Umläufen Netz- und Steuergeräte auf, die zur Steuerung eines abnehmenden Erregerstroms eine nichtlineare Rampe mit abgeflachtem Verlauf am Ende der Rampe vorsehen. Dadurch werden in vorteilhafter Weise die Injektionsmittel für das Synchrotron, für die so genannte Multiturn-Injektion zuverlässiger gestaltet, indem eine nicht-lineare, z.B. parabolische Rampe für die Bumper-Magnetfelder mit einem steilen Abfall am Beginn der Rampe und mit abflachendem Verlauf am Ende der Rampe vorgesehen wird und die Bahnverlagerung mit höchstens drei Bumpermagneten an Stelle der bekannten Anordnung mit vier Bumpermagneten zuverlässig und präzise erreicht wird, wobei kein Bumpermagnet in der sehr eng gefüllten Injektionsstrecke benötigt wird.

Ferner wird eine optimale Geometrie für das elektrostatische Injektionsseptum mit Strahleintritt im Zentrum der Apertur und Strahlaustritt an der Innenelektrode des Septums, sowie mit präziser Strahljustierung durch Einstellung der zwei Parameter Ablenkspannung am elektrostatischen Injektionsseptum und automatischer Justierung für den Eintrittswinkel des injizierten Ionenstrahls am Eintritt in das Injektionsseptum in einer weiteren bevorzugten Ausführungsform erreicht, indem die Injektionsmittel ein elektrostatisches Injektionsseptum aufweisen, dessen gekrümmte elektrostatische Ablenkplatten einen größeren Krümmungsradius aufweisen als der Bahnradius des vorbeschleunigten injizierten und abgelenkten Ionenstrahls.

In einer weiteren bevorzugten Ausführungsform der Erfindung weisen die Extraktionsmittel für die Anregung der nicht-linearen Resonanz zur präzisen Justierung der Separatrix und entsprechend des Austrittswinkels für den extrahierten Strahl sechs einzeln einstellbare Sextupolmagnete strahlaufwärts vor jeder gebogenen Strahlstrecke mit Dipolmagnetpaar als elektronisch exakt steuerbare Extraktionsmittel auf. Dazu sind die Erregerströme der einzelnen Sextupolmagnete für die Resonanzextraktion einstellbar, und die Sextupolmagnete stehen mit einem fixierten elektrostatischen Extraktionsseptum als eines der Extraktionsmittel für die Extraktion des Ionenstrahls in Wirkverbindung. Ferner wird mit einem elektrostatischen Extraktionsseptum sowie mit nur einem Septummagneten für die Strahlauslenkung an Stelle von zwei Septummagneten sowie mit optimierter technischer Auslegung der Biege- und Quadrupolmagnete im Synchrotron eine hohe Zuverlässigkeit in Bezug auf den Extraktionswinkel erreicht.

Vorzugsweise weist der Teilchenbeschleuniger als eine der Ionenquellen mindestens eine Laser-Ionenquelle für die Erzeugung von Strahlimpulsen von Kohlenstoff-Ionen auf. Mit einer derartigen Ionenquelle können Ionenstrahlen erzeugt werden, die vorzugsweise Kohlenstoff-Ionen mit dem Ladungszustand q=4 (C⁴⁺-Ionen) aufweisen. Diese Laser-Ionenquelle hat gegenüber anderen Ionenquellen die Vorteile:
(a) hohe Strahlintensitäten von mehr als 1·10¹⁰ C⁴⁺-Ionen in kurzen Strahlpulsen von 20-30 µs Dauer,
(b) lange Standzeiten von vielen Wochen ohne Service,
(c) hohe Zuverlässigkeit über viele Betriebsjahre, und
(d) günstige Investitions- und Betriebskosten.

In einer weiteren bevorzugten Ausführungsform der Erfindung weist der Teilchenbeschleuniger als Injektor-Linearbeschleuniger einen Linearbeschleuniger mit IH-Sektionsmodulen und außerhalb von Vakuumanlagen der IH-Sektionsmodule Quadrupol-Linsenmodule auf. Ein derartiger Linearbeschleuniger hat gegenüber der bekannten Beschleunigungskammer zur linearen Vorbeschleunigung folgende Vorteile:
(a) modularer Aufbau des Linearbeschleunigers mit drei kurzen Beschleunigerabschnitten von 1,5 m- 2 m Länge für die so genannte IH-Sektion,
(b) modularer Aufbau der Hochfrequenz-Anlagen mit HF-Generatoren von maximal 180 kW HF-Leistung mit entsprechenden Vereinfachungen gegenüber Anlagen in der bisher üblichen Leistungsklasse 1-2 MW bis2 MW,
(c) technisch einfacher und für den Service vorteilhafter Einbau der Quadrupol-Linsen zwischen den Beschleuniger-Abschnitten außerhalb der Vakuumanlage, z.B. durch mechanische Teilung des Quadrupoljochs in einer Symmetrieebene
(d) hohe Zuverlässigkeit über viele Betriebsjahre, und
(e) günstige Investitions- und Betriebskosten.

Weiterhin ist es vorgesehen, dass zwischen Bestrahlungsplätzen und sechszähligem Synchrotronring Strahlführungen angeordnet sind, die eine Kompensation der horizontalen Dispersion direkt nach dem Synchrotronring und für die Verteilung durch vertikale Ablenkung auf die verschiedenen Bestrahlungsplätze aufweisen. Damit wird in vorteilhafter Weise eine hohe Stabilität der Strahlposition an den Bestrahlungsplätzen erreicht, wobei mit Hilfe der ausschließlich vertikaler Ablenkung, mehrere Bestrahlungsplätze mit unterschiedlichen Einfallswinkeln α mit 0 ≤ α ≤ 90° versorgt werden, wobei 0° ein horizontal eintreffender Einfallwinkel und 90° ein senkrechter von oben eintreffender Einfallswinkel α ist.

Die Erfindung wird nun an Hand der beigefügten Figuren näher erläutert.
- Figur 1: zeigt schematisch eine Draufsicht auf einen sechs- zähligen Synchrotronring eines Teilchenbeschleuni- gers einer Ausführungsform der Erfindung;
- Figur 2: zeigt schematisch einen teilweisen Querschnitt durch einen Dipolmagneten eines Dipolmagnetpaares des sechszähligen Synchrotronrings gemäß Figur 1;
- Figur 3: zeigt schematisch einen teilweisen Querschnitt durch einen Quadrupolmagneten des sechszähligen Synchrotronrings gemäß Figur 1;
- Figur 4: zeigt schematisch ein Diagramm der horizontalen und vertikalen Strahlradien (Strahlenveloppen) im Synchrotronring gemäß Figur 1;
- Figur 5: zeigt eine schematische Draufsicht auf ein elekt- rostatisches Injektionsseptum;
- Figur 6: zeigt schematisch ein Diagramm der Bahnverlagerung des Ionenstrahls im Synchrotronring gemäß Figur 1 unter Einfluss von drei Bumpermagneten im Strah- lengang;
- Figur 7: zeigt schematisch ein Diagramm einer von Umlauf zu Umlauf des Ionenstrahls radialen Akzeptanz ausge- löst von Bumpermagneten und eine parabolische Ram- pe für die Magnetfelder der Bumpermagnete;
- Figur 8: zeigt schematisch eine Draufsicht auf einen Aus- schnitt eines sechszähligen Synchrotronrings mit dem Extraktionszweig;
- Figur 9: zeigt schematisch ein Diagramm mit einer Aus- trittsrichtung des extrahierten Ionenstrahls mit
- Figur 10: Hilfe der sechs einzeln einstellbaren Sextupol- magnete; zeigt schematisch ein Diagramm mit mehreren unter- schiedlichen Austrittsrichtungen des extrahierten Ionenstrahls mit Hilfe der sechs einzeln einstell- baren Sextupolmagnete;
- Figur 11: zeigt schematisch ein Diagramm der Strahlauslen- kung im Synchrotronring im Bereich eines Extrakti- onsmittels;
- Figur 12: zeigt schematisch eine Draufsicht auf einen Teil- chenbeschleuniger mit Ionenquelle, Injektor- Linearbeschleuniger, sechszähligen Synchrotronring und Extraktionszweig einer Ausführungsform der Er- findung;
- Figur 13: zeigt schematisch eine Prinzipskizze einer Ionen- quelle;
- Figur 14: zeigt schematisch eine Prinzipskizze eines Injek- tor-Linearbeschleunigers;
- Figur 15: zeigt schematisch eine Seitenansicht einer Strahl- führung zu mehreren Bestrahlungsräumen.

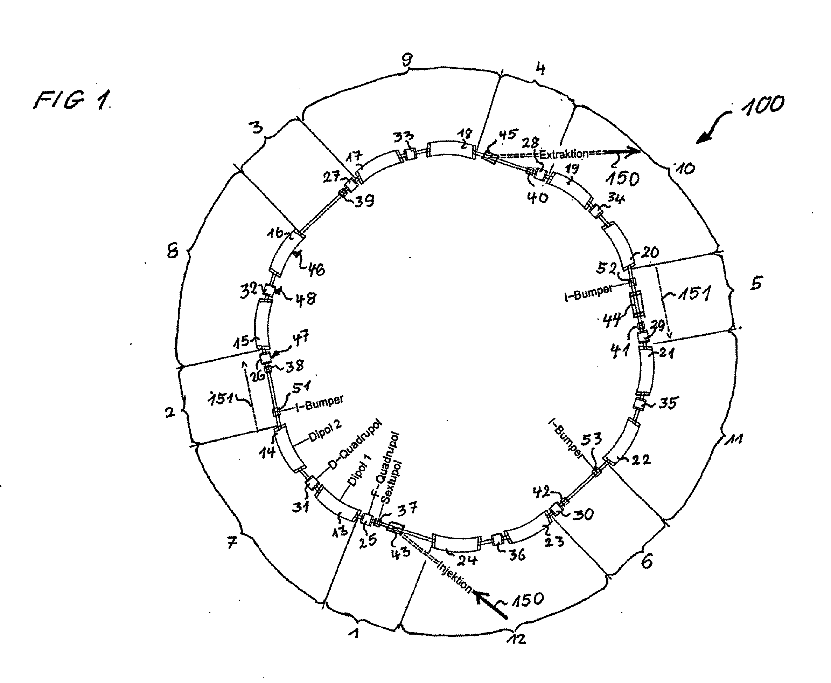

Figur 1 zeigt schematisch eine Draufsicht auf einen sechszähligen Synchrotronring 100 eines Teilchenbeschleunigers einer Ausführungsform der Erfindung. Dazu weist der sechszählige Synchrotronring 100 sechs geradlinige Strahlstrecken 1 bis 6 und sechs gebogene Strahlstrecken 7 bis 12 auf. Von den sechs geradlinigen Strahlstrecken 1 bis 6 sind auf einer ersten geradlinigen Strahlstrecke 1 Injektionsmittel 43 für das Einführen eines linearbeschleunigten Ionenstrahls 150 in den Synchrotronring 100 angeordnet. Im Verlauf einer zweiten geradlinigen Strahlstrecke 5 ist mindestens ein Beschleunigungsmittel 44 für den Ionenstrahl 150 vorhanden. An einer dritten geradlinigen Strahlstrecke 4 sind Extraktionsmittel 45 für das Extrahieren des nach mehreren Umläufen in Strahlrichtung 151 hochbeschleunigten Ionenstrahls 150 vorgesehen.

Dazu weist jede gebogene Strahlstrecke 7 bis 12 ein Dipolmagnetpaar 13/14, 15/16, 17/18, 19/20, 21/22 und 23/24 auf. Zwischen den beiden Dipolmagneten eines Dipolmagnetpaares 13/14, 15/16, 17/18, 19/20, 21/22 bzw. 23/24 ist ein horizontal defokussierender Quadrupolmagnet 31 bis 36 angeordnet. Strahlaufwärts vor jedem Dipolmagnetpaar 13/14, 15/16, 17/18, 19/20, 21/22 und 23/24 ist ferner ein horizontal fokussierender Quadrupolmagnet 25 bis 30 vorgesehen. Somit weist das Synchrotron eine optimale Anordnung von Dipolmagneten 13 bis 24 als Biegemagnete 46 und Quadrupolmagneten 25 bis 36 auf. In dieser Anordnung wechseln sich Dipolmagnetpaare 13/14, 15/16, 17/18, 19/20, 21/22 und 23/24 als Biegemagnetpaare und Quadrupolmagnete in einer Struktur F (Fokussiermagnet 47), BM (Biegemagnet 46), D (Defokussiermagnet 48) und BM (Biegemagnet 46) in sechs Superperioden als gebogene Strahlstrecken 7 bis 12 mit sechs freien geradlinigen Strahlstrecken 1 bis 6 ab.

Für das Synchrotron wird somit ein optimiertes Magnetsystem mit 12 leichten Dipolmagneten 13 bis 24, die als Kombination von 'window-frame' und H-Magnet ausgelegt sind, eingesetzt. Dieses Magnetsystem hat gegenüber anderen Konzepten folgende Vorteile:
(a) Reduktion des Gesamtgewichts aller Magnete beispielsweise auf zusammen weniger als 100 t im Vergleich zu mehr als 210 t im Stand der Technik bei vergleichbaren Randbedingungen der Systeme für die injizierte und extrahierte Ionenstrahlenergie,
(b) maximale Magnetgewichte der Einzelmagnete von höchstens 5 t und für ein Biegemagnetpaar von höchstens 10 t und entsprechend einfache Montage und Demontage im Vergleich zu mehr als 25 t für einen einzelnen Biegemagneten im Stand der Technik bei vergleichbaren Randbedingungen der Systeme, womit eine deutliche Gewichts- und Kostenreduzierung verbunden ist,
(c) erhebliche Verringerung der erforderlichen Pulsleistung für die Magnetstromversorgungen aufgrund der nun möglichen geringeren Apertur der Biegemagnetpaare gegenüber einzelnen Biegemagneten und entsprechend günstigere Kosten für den Aufbau und Betrieb des Teilchenbeschleunigers.

Diese Vorteile werden durch den Einsatz modifizierter Magnetkonzepte für die Dipolmagnete 13 bis 24 und Quadrupolmagnete 25 bis 36 wie sie in den nachfolgenden Figuren dargestellt werden, sowie durch die Wahl einer anderen Magnetanordnung mit zwölf Dipolmagneten 13 bis 24 und zwölf Quadrupolmagneten 25 bis 36, wie es Figur 1 zeigt, erreicht.

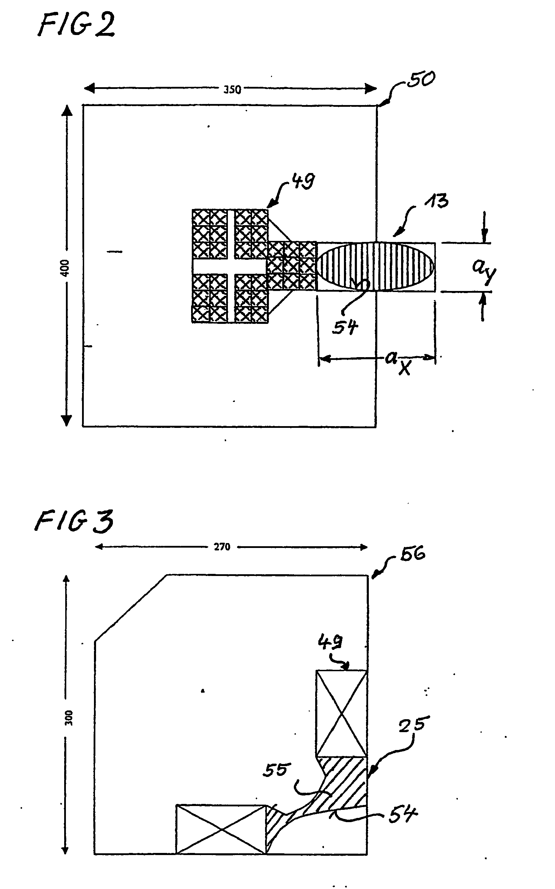

Figur 2 zeigt schematisch einen teilweisen Querschnitt durch einen Dipolmagneten 13 eines Dipolmagnetpaares 13/14 bzw. Biegemagnetpaares des sechszähligen Synchrotronrings 100 gemäß Figur 1, wobei lediglich eine spiegelbildliche Hälfte 50 des Dipolmagneten 13 gezeigt wird. Die Maßzahlen sind beispielhaft in Millimetern angegeben. Das von Polschuhen umschlossene elliptische Aperturprofil 54 und die Magnetspulenkonfiguration 49 sind kennzeichnend für diese Kombination von 'window-frame' und H-Magnet-Typ, der nur aufgrund der erfindungsgemäßen verkürzten gebogenen Strahlstrecken pro Dipolmagnet des Synchrotronringes realisiert werden kann. Eine optimale Auslegung des Dipolmagneten 13 in Bezug auf die erforderlichen Magnetaperturen aₓ in horizontaler Richtung und a_{y} in vertikaler Richtung im Synchrotron ist durch die erfindungsgemäße optimierte technische Auslegung der Biege- und Quadrupolmagnete realisiert.

Figur 3 zeigt schematisch einen teilweisen Querschnitt durch einen Quadrupolmagneten 25 des sechszähligen Synchrotronrings 100 gemäß Figur 1. Dabei zeigt Figur 3 lediglich einen Quadranten 56 des Quadrupolmagneten im Querschnitt. Die Maßzahlen sind beispielhaft in Millimetern angegeben. Dieser schematische Querschnitt verdeutlicht die Auslegung des Quadrupolmagneten 25 mit Rechteck-Profil und entsprechend geringer Gesamtbreite. Die Magnetspulenkonfiguration 49 und die Polschuhkonfiguration 55 unterscheiden sich von der des Dipolmagneten 13 gemäß Figur 2 und sind in Bezug auf Gewicht und auch in Bezug auf die gespeicherte Energie und auf den Energieverbrauch beim Betrieb des Synchrotrons optimiert..

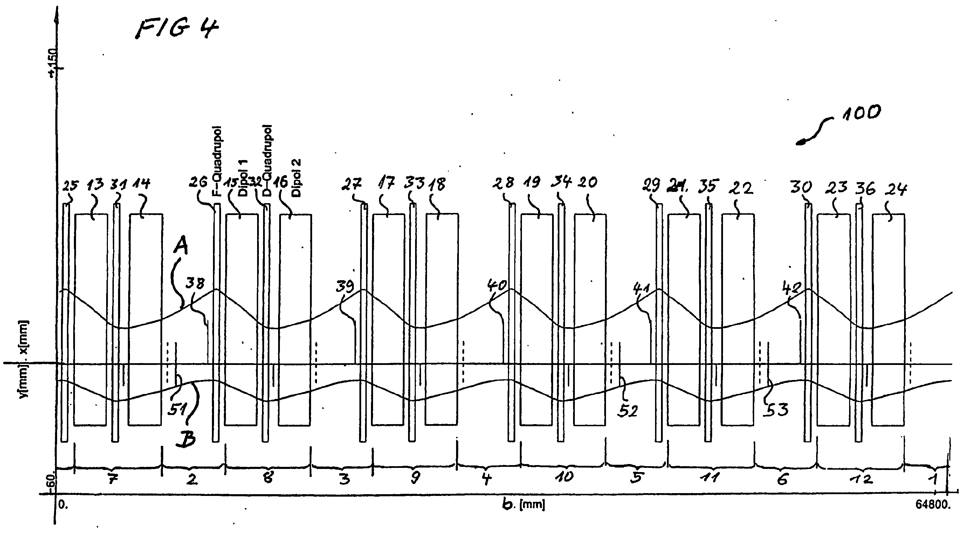

Figur 4 zeigt schematisch ein Diagramm mit horizontalen Strahlradien in x-Richtung mit Kurve A und mit vertikalen Strahlradien in y-Richtung mit Kurve B, die jeweils in Millimetern auf der Ordinate des Diagramms abgebildet sind. Komponenten mit gleichen Funktionen wie in den vorhergehenden Figuren werden mit gleichen Bezugszeichen gekennzeichnet und nicht extra erörtert. Entlang der Abszisse des Diagramms ist die Bahnlänge b in Millimetern im Synchrotronring dargestellt. Die Strahlauslenkungen in x- und y-Richtung in Millimetern sind bei vergleichbaren Randbedingungen gegenüber bekannten Synchrotronringen deutlich geringer, so dass in vorteilhafter Weise kleinere Aperturdimensionen aₓ und a_{y}, wie in Figur 2 gezeigt, mit dieser Erfindung erreicht werden.

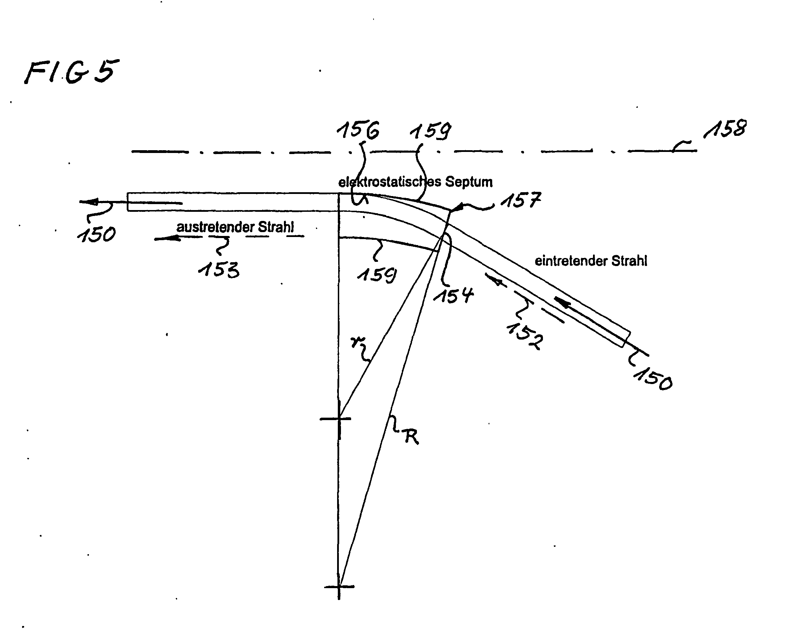

Figur 5 zeigt eine schematische Draufsicht auf ein elektrostatisches Injektionsseptum 157, das zum erfindungsgemäßen Injektionssystem für das Synchrotron gehört. Die Strichpunktierte Linie 158 zeigt die Lage des Bahnzentrums einer gradlinigen Strahlstrecke, in das mit Hilfe der in Figur 1 gezeigten Injektionsmittel 43 ein Ionenstrahl 150 im Mehrfachumlaufverfahren auch "multi-turn-injection" genannt, injiziert werden soll. Das erfindungsgemäße Injektionsseptum 157 ist so auslegt, dass ein reproduzierbarer Betrieb mit minimalen Strahlverlusten automatisch eingestellt werden kann. Dazu weist das elektrostatische Injektionsseptum 157 eine optimale Geometrie mit Strahleintritt 154 im Zentrum der Apertur des elektrostatisches Injektionsseptum 157 für den eintretenden Ionenstrahl 152 und Strahlaustritt 155 an der Innenelektrode 156 des Injektionsseptums 157 für den austretenden Ionenstrahl 153 und eine präzise Einstellung von Strahllage und Strahlwinkel am Strahlaustritt 155 wird durch Einstellen von zwei Parametern, nämlich Ablenkspannung am elektrostatischen Injektionsseptum 157 und Eintrittswinkel des injizierten Ionenstrahls 150 am Strahleintritt 154 in das Injektionsseptum 157 erreicht. Dazu weist das elektrostatische Injektionsseptum 157 gekrümmte elektrostatische Ablenkplatten 159 auf, deren Krümmungsradien R größer sind als der Bahnradius r des vorbeschleunigten injizierten und abgelenkten Ionenstrahls 150.

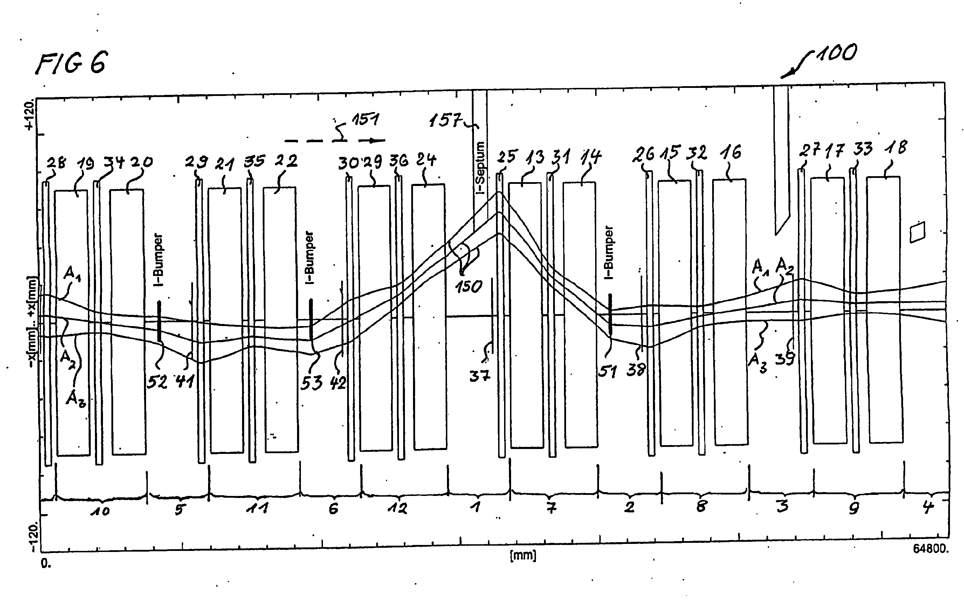

Figur 6 zeigt schematisch ein Diagramm der Bahnverlagerungen des Ionenstrahls 150 im Synchrotronring 100 gemäß Figur 1 unter Einfluss von drei Bumpermagneten 51, 52 und 53 im Strahlengang. Diese Bahnverlagerung mit der Strahlenveloppe A₁, A₃ um das Strahlzentrum A₂ kommt mit drei schnellen Ferritmagneten zum Aufbau einer lokalen Bahnstörung außerhalb der Injektionsstrecke 1 im Synchrotron aus, anstelle der aus dem Stand der Technik bekannten Anordnung mit einem der drei Bumpermagnete in der Injektionsstrecke 1. In diesem Beispiel werden zwei Bumpermagnete 52 und 53 in den geraden Strecken 5 und 6 in Strahlrichtung 151 vor der Injektionsstrecke 1 mit dem Injektionsseptum 157 und ein Bumpermagnet 51 nach der geradlinigen Injektionstrecke 1 eingesetzt, so dass in vorteilhafter Weise die mit dem in Figur 5 gezeigten Injektionsseptum 157, dem Sextupolmagneten 37 und dem horizontal fokussierenden Quadrupolmagneten 25 eng gefüllte Injektionsstrecke 1 frei von Bumpermagneten bleibt. In einem weiteren Optimierungsschritt können gegebenenfalls die zwei ersten Bumpermagnete 52 und 53 durch einen einzigen Bumpermagneten ersetz werden.

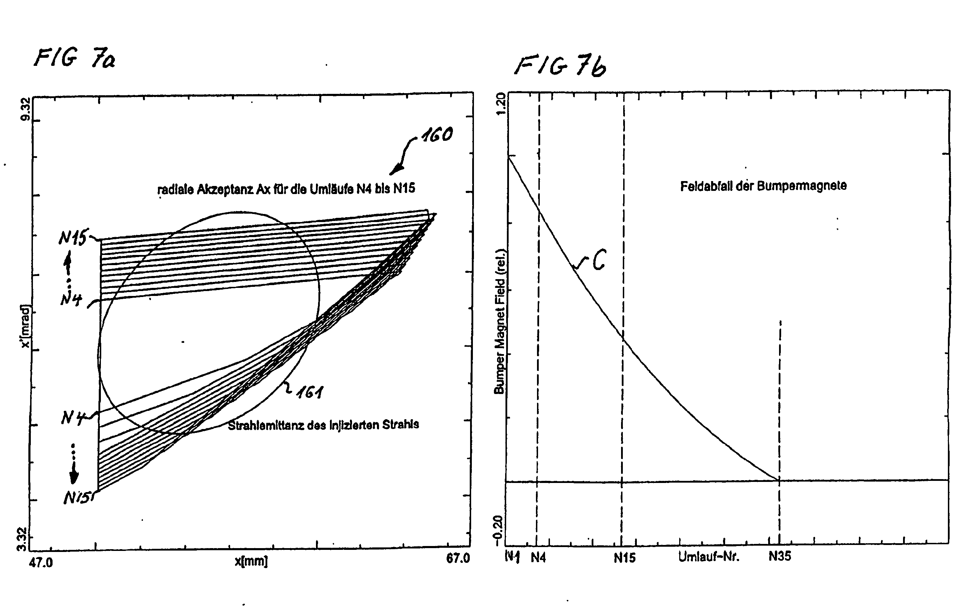

Figur 7 zeigt mit Figur 7a schematisch ein Diagramm des Phasenraums 160 einer von Umlauf zu Umlauf (N4 bis N15) des Ionenstrahls sich verbessernden radialen Akzeptanz, wobei auf der Ordinate die Winkelkoordinaten des Phasenraums 160 in mrad angegeben sind und auf der Abszisse die Ostskoordinaten x in mm gezeigt werden. Die Ellipse 161 zeigt die erreichbare optimale Anpassung der Strahlemittanz des injizierten Ionenstrahls und die von Umlauf zu Umlauf N4 bis N15 variable Akzeptanz für die Multiturn-Injektion.

Diese radiale Akzeptanz wird von den drei Bumpermagneten 51, 52 und 53 wie sie in Figur 6 gezeigt werden mittels einer in Figur 7b gezeigten parabolischen Rampe C für die Magnetfelder der Bumpermagnete ausgelöst. Das Diagramm in 7b zeigt die relative Stärke des Bumpermagnetfelds der Bumpermagnete 51, 52 und 53 auf der Ordinate und die Zahl der Umläufe N1 bis N35 auf der Abszisse. Die parabolische Rampe C für die Magnetfelder der Bumpermagnete weist zu Beginn einen steilen Abfall und einen abflachenden Verlauf am Ende der Rampe C auf.

Das in den Figuren 5, 6 und 7 beschriebene Injektionssystem für den erfindungsgemäßen Teilchenbeschleuniger weist nachfolgende Vorteile auf:
(a) optimaler Aufbau bei minimalen Kosten,
(b) hoher Wirkungsgrad für die multi-turn-injection von etwa 85%, d.h. minimale Strahlverluste bei Injektion und entsprechend minimale radioaktive Belastung, so dass die Bedingungen der Strahlenschutzverordnungen mit diesem erfindungsgemäßen Synchrotronring 100 im Gegensatz zu Zyklotronbeschleunigern erfüllt sind,
(c) sichere, reproduzierbare Einstellverfahren, die weitgehend automatisiert sind.
Dazu sind die erfindungsgemäßen Injektionsmittel 43 für die so genannte multi-turn-injection, in folgender Weise verbessert:
(a) nicht-lineare, z.B. parabolische Rampe C für die Bumper-Magnetfelder mit einem steilen Abfall am Beginn der Rampe C und mit abflachendem Verlauf am Ende der Rampe C;
(b) Bahnverlagerung durch ein multi-turn-injection-system mit drei optimal angeordneten so genannten Bumpermagneten 51, 52 und 53, wobei zwei dieser Magnete 52 und 5.3 in den zwei geradlinigen Strecken 5 und 6 vor der Injektionsstrecke 1 den Ionenstrahl 150 auslenken und ein dritter Bumpermagnet 51 in der geradlinigen Strecke 2 nach der Injektionsstrecke 1 den Ionenstrahl wieder einlenkt;
(c) Anstelle der bekannten Anordnung mit drei Bumpermagneten, wobei einer in der Injektionsstrecke 1 angeordnet ist, nur noch zwei oder höchstens drei Bumpermagnete wobei in der sehr eng gefüllten geradlinigen Strahlstrecke 1 mit Injektionsmitteln 43 keiner der Bumpermagnete 51, 52 und 53 eingesetzt wird;
(d) optimale Geometrie für das elektrostatische Injektionsseptum 157 mit Strahleintritt 154 im Zentrum der Apertur und Strahlaustritt 155 an der Innenelektrode 156 des Injektionsseptums 157, sowie mit präziser Strahljustierung durch Einstellung der zwei Parameter Ablenkspannung am elektrostatischen Injektionsseptum 157 und möglichst automatische Justierung für den Eintrittswinkel des injizierten Ionenstrahls am Eintritt in das Injektionsseptum 157.

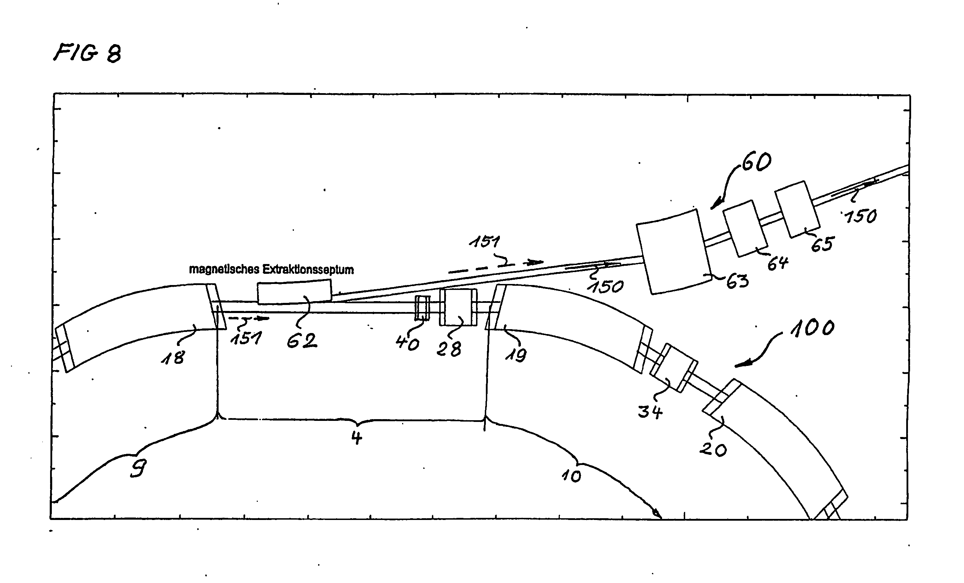

Figur 8 zeigt schematisch eine Draufsicht auf einen Ausschnitt eines sechszählige Synchrotronrings 100 mit dem Extraktionszweig 60, der von einer geradlinigen Strahlstrecke 4 bzw. Extraktionsstrecke 4 abzweigt. Die Strahlauslenkung in der Extraktionsstrecke 4 weist nur einen einzigen Extraktionsseptummagneten 62 auf, da die Dimensionen des Dipolmagneten 19 des Dipolmagnetpaares 19/20 aufgrund des erfindungsgemäßen Synchrotronringes 100 derart vermindert werden, dass unter einem flacheren Winkel die Extraktion des Ionenstrahls 150 erfolgt, im Gegensatz zu Synchronringen des Standes der Technik, bei denen mindestens zwei Septummagnete erforderlich sind, um mit einem größeren Auslenkwinkel an den nachfolgenden größeren Dimensionen der im Stand der Technik eingesetzten Dipolmagnete vorbei zu kommen.

Das elektromagnetische Extraktionsseptum 62 kann derart angeordnet sein, dass es den extrahierten Ionenstrahl 150 in einen horizontal umlenkenden Dipolmagneten 63 einkoppelt, der den Ionenstrahl 150 an zwei strahlabwärts angeordnete Quadrupole 64 und 65 auf dem Extraktionszweig 60 liefert, die zu einer hochenergetischen Ionenstrahlführung gehören. Das Extraktionssystem weist neben dem Extraktionsseptummagneten 62 ein elektrostatisches Extraktionsseptum auf, das strahlaufwärts von der Extraktionsstrecke 4 in der gradlinigen Strahlstrecke 3 angeordnet ist. Ferner sind für die Anregung einer nicht-linearen Resonanz für die Extraktion in jeder der geradlinigen Strahlstrecken 1 bis 6 Sextupole 37 bis 42 angeordnet.

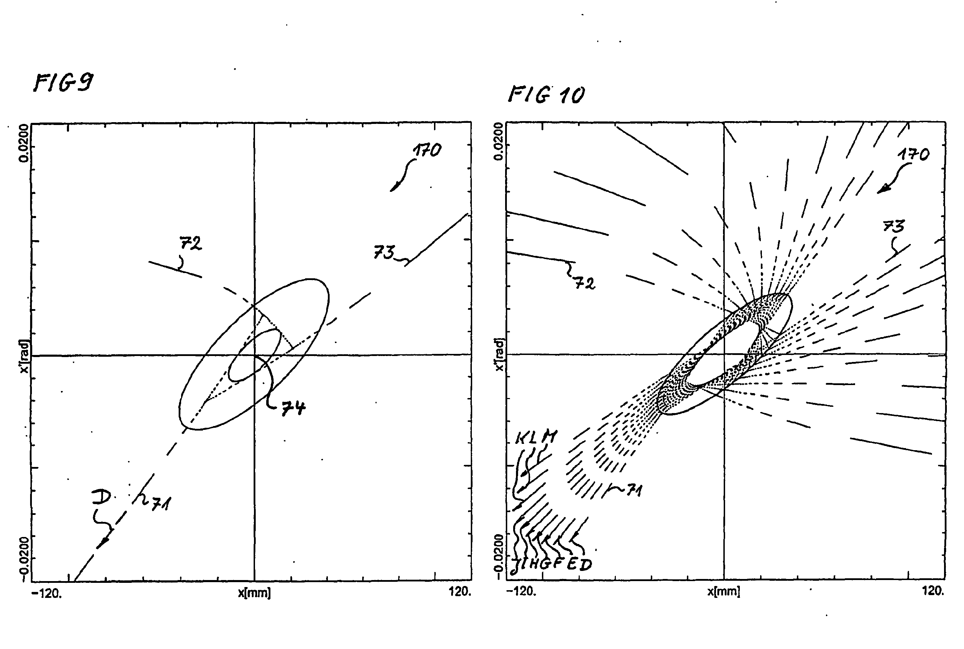

Figur 9 zeigt schematisch ein Diagramm eines einzelnen Austrittsstrahls 71 des extrahierten Ionenstrahls, dessen Richtung D im Phasenraum mit Hilfe einer durch die sechs in Figur 1 gezeigten Sextupole 37 bis 42 erreichten Anregung einer nicht-linearen Resonanz eingestellt werden kann. Dazu zeigt Figur 9 eine Darstellung eines Phasenraums 170, wobei die Winkelkoordinate x' auf der Ordinate des Diagramms gezeigt wird, und die Ortskoordinate x auf der Abszisse der Darstellung zu sehen ist.

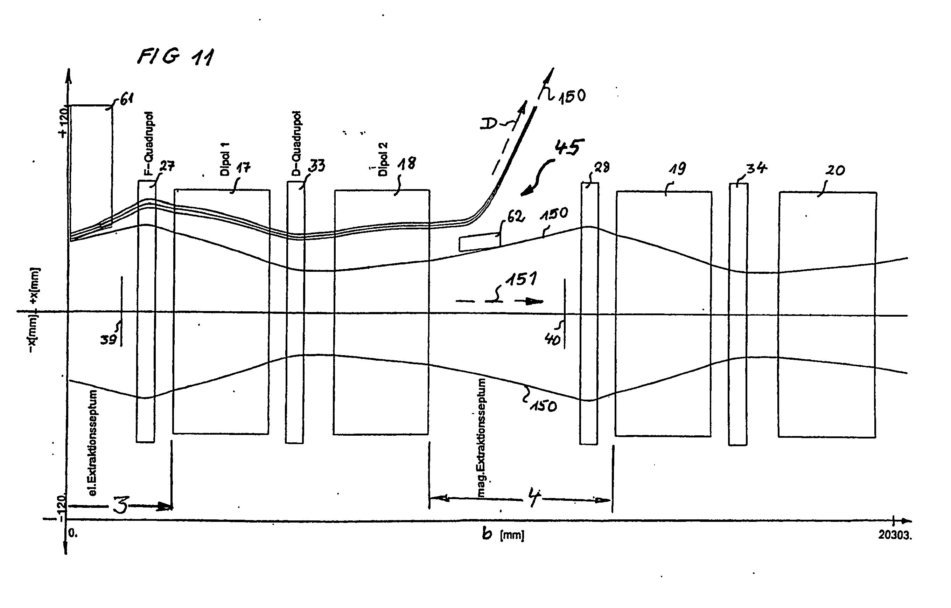

Während der Resonanzextraktion werden die Ionen instabil und machen bei der Darstellung im Phasenraum 170 für die Bewegung in der Horizontalebene mit jedem Umlauf einen Schritt von einem der drei dargestellten Armen 71, 72, 73 zum nächsten. Sie pendeln bei Betrachtung der Ortskoordinate x um die zentrale Sollbahn 74 bis sie im letzten Schritt auf dem linken unteren Arm 71 in das in Figur 11 gezeigte elektrostatische Extraktionsseptum 61 eintreten. Durch die präzise Justierung der Separatrix kann entsprechend die Austrittsrichtung D für den extrahierten Ionenstrahl mit Hilfe von sechs einzeln einstellbaren Sextupolmagneten und der optimale Wirkungsgrad für die Resonanzextraktion eingestellt werden. Auf diese Weise wird die aufwendige und umständliche mechanischgeometrische Justierung des in Figur 11 gezeigten elektrostatischen Extraktionsseptums 61 vermieden.

Figur 10 zeigt schematisch ein Diagramm für mehrere mit Hilfe der sechs einzeln einstellbaren Sextupolmagnete einstellbarer Austrittsrichtungen D bis M des extrahierten Ionenstrahls im Phasenraum 170.

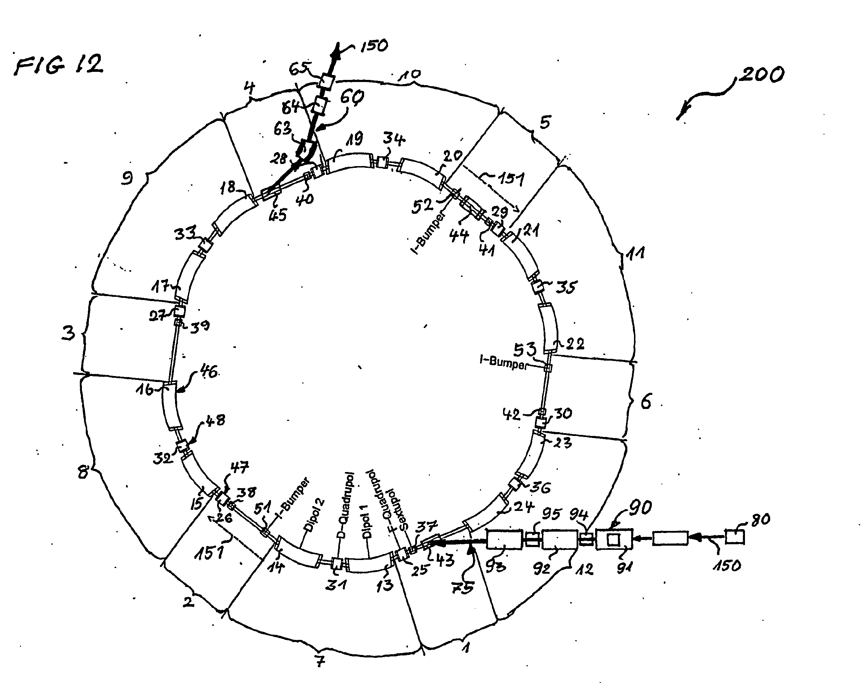

Figur 11 zeigt schematisch ein Diagramm der Strahlauslenkung im Synchrotronring im Bereich eines Extraktionsmittels 45. Komponenten mit gleichen Funktionen, wie in den vorhergehenden Figuren werden in den nachfolgen Figuren 12 bis 15 mit gleichen Bezugszeichen gekennzeichnet und nicht extra erörtert. Auf der Abszisse ist wieder die Bahnlänge b in Millimetern und auf der Ordinate ist jedoch ausschließlich die Auslenkung in x- Richtung in Millimetern aufgetragen. Zur Extraktion wird mit Hilfe der sechs Sextupolmagnete, von denen die Sextupolmagnete 39 und 40 des Synchrotronringes hier zu sehen sind, eine nichtlineare Extraktionsresonanz erzeugt. Ein elektrostatisches Extraktionsseptum 61 ist strahlaufwärts eines elektromagnetischen Extraktionsseptums 62 in der geradlinigen Strahlstrecke 3 angeordnet. Die Anregung einer Extraktionsresonanz wurde bereits oben beschrieben, wobei mit Hilfe der elektrisch und damit automatisch einstellbaren Sextupolmagnete 37 bis 42 die Austrittsrichtung D des Ionenstrahls 150 präzise definiert werden kann.

Figur 12 zeigt schematisch eine Gesamtansicht eines Teilchenbeschleuniger 200 mit Ionenquelle 80, Injektor-Linearbeschleuniger 90, sechszähligem Synchrotronring 100 Injektionszweig 75 und Extraktionszweig 60 einer Ausführungsform der Erfindung.

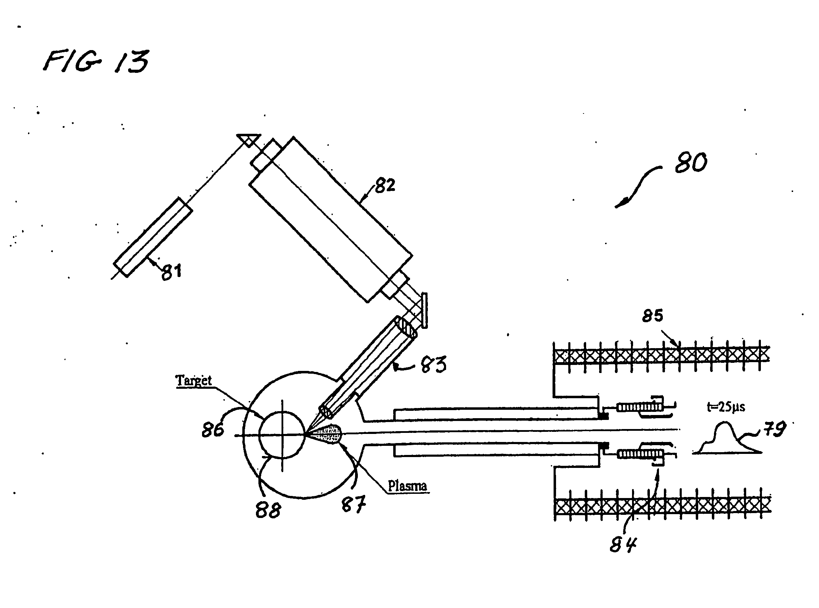

Figur 13 zeigt schematisch eine Prinzipskizze einer Ionenquelle 80. Als Ionenquelle wird eine Laser-Ionenquelle eingesetzt, die einen HeNe-Laser 81 aufweist, der seinerseits einen CO₂-Laser 82 anregt. Über eine Objektiv 83 wird dann der Laserstrahl auf die Oberfläche 88 eines Kohlenstoffziel bzw. Targets 86 gerichtet, wodurch die Oberfläche 88 des Kohlenstoffziels zu einem elektrisch geladenen Plasma 87 zerstäubt wird. Dieses Plasma 87 wird in einem Vorbeschleuniger 85 beschleunigt.

Diese Laserionenquelle 80 ist für die Erzeugung von sehr kurzen Strahlimpulsen 79 von Kohlenstoff-Ionen kleiner gleich 30 µs bei hoher Strahlintensität besonders geeignet. Für die Erzeugung von Ionenstrahlen, vorzugsweise Kohlenstoff-Ionen mit dem Ladungszustand q=4 (C⁴⁺-Ionen) bieten Laser-Ionenquellen 80 gegenüber anderen Ionenquellen wichtige Vorteile:
(a) hohe Strahlintensitäten von mehr als 1x10¹⁰ C⁴⁺-Ionen in kurzen Strahlimpulsen 79, vorzugsweise von 20 µs bis 30 µs Dauer;
(b) lange Standzeiten von vielen Wochen ohne Service;
(c) hohe Zuverlässigkeit über viele Betriebsjahre, und
(d) günstige Investitions- und Betriebskosten.

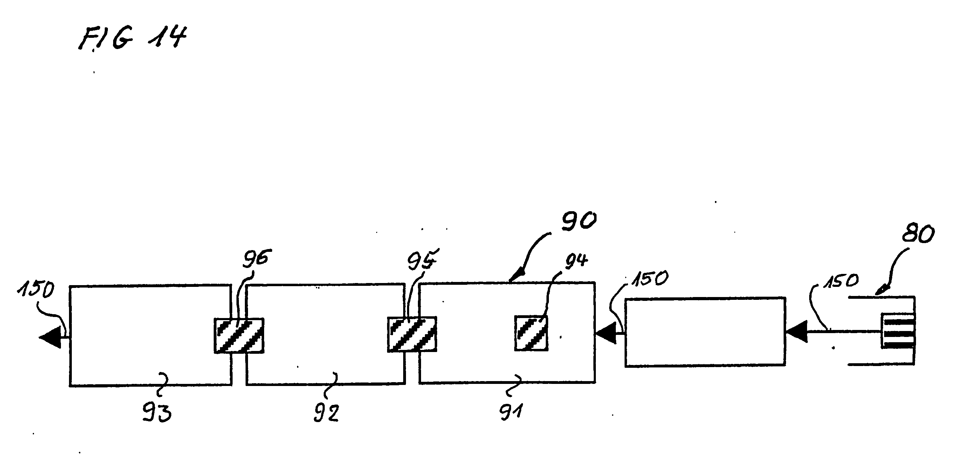

Figur 14 zeigt schematisch eine Prinzipskizze eines Injektor-Linearbeschleunigers 90. Der Linearbeschleuniger 90 weist einen modularen Aufbau mit so genannten IH-Sektionen 91 bis 93 auf. Dazu sind drei Quadrupol-Tripletts als Quadrupol-Linsen 94 bis 96 teilweise zwischen den IH-Sektionen 91, 92 und 93 angeordnet. Der modulare Aufbau der Hochfrequenz-Anlagen aus IH-Sektionen 91 bis 93, wird mit HF-Generatoren von maximal 180 kW HF-Leistung realisiert. Durch die Anordnung der außerhalb der Vakuumanlage zwischen den drei Beschleunigerabschnitten in Form der IH-Sektionen 91, 92 und 93 angeordneten Quadrupol-Linsen 95 und 96 ist ein einfacher und Service freundlicher Zusammenbau des Linearbeschleunigers möglich.

Dieser Linearbeschleuniger 90 einer bevorzugten Ausführungsform der Erfindung hat somit folgende Vorteile:
(a) modularer Aufbau des Linearbeschleunigers 90 mit drei kurzen Beschleunigerabschnitten von 1,5 m bis 2 m Länge für jede so genannte IH-Sektion 91, 92 und 93,
(b) modularer Aufbau der Hochfrequenz-Anlagen mit HF-Generatoren von maximal 180 kW HF-Leistung mit entsprechenden Vereinfachungen gegenüber Anlagen in der Leistungsklasse 1 MW bis 2 MW,
(c) technisch einfacher und für den Service vorteilhafter Einbau der Quadrupol-Linsen zwischen den BeschleunigerAbschnitten außerhalb der Vakuumanlage zumindest für die Quadrupol Triplets 95 und 96.

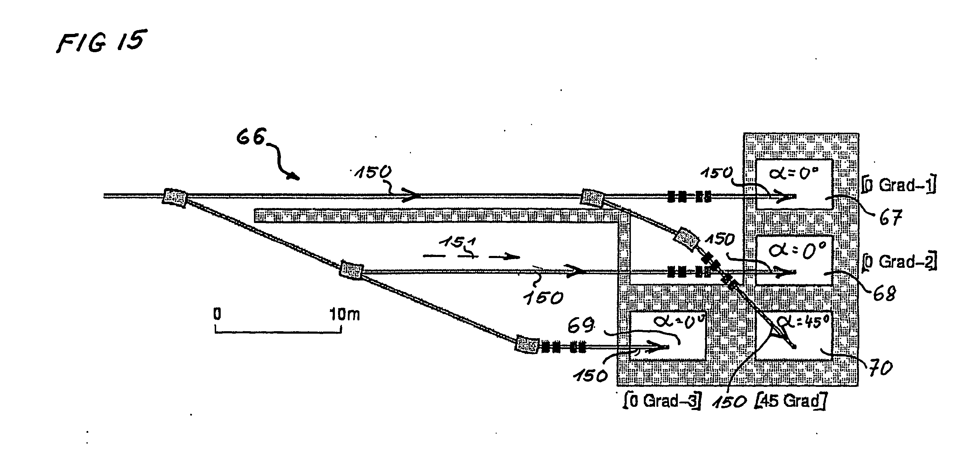

Figur 15 zeigt schematisch eine Seitenansicht einer vertikal ausgelenkten Strahlführung 66 zu mehreren Bestrahlungsräumen mit Bestrahlungsplätzen 67 bis 70. Die Strahlführung 66 kann zwischen Synchrotron und Bestrahlungsplätzen 67 bis 70 mit einem horizontaler Ablenkung direkt nach dem Synchrotron arbeiten oder über eine separate vertikale Ablenkung zur Verteilung des Ionenstrahls 150 auf die Bestrahlungsplätze 67 bis 70 verfügen. Dazu ist die Strahlführung 66 zwischen Synchrotron und Bestrahlungsplätzen 67 bis 70 für die Therapie wahlweise mit einer Kompensation der horizontalen Dispersion direkt nach dem Synchrotron oder für eine Verteilung auf die verschiedenen Bestrahlungsplätze 67 bis 70 mit einer vertikalen Ablenkung für eine separate unabhängige Kompensation der vertikalen Dispersion ausgestattet. Damit wird vorteilhaft eine hohe Stabilität der Strahlposition an den Bestrahlungsplätzen 67 bis 70 erreicht. In Figur 15 ist diese Anordnung am Beispiel einer Strahlführung 66 für vier Bestrahlungsplätze 67 bis 70 dargestellt, wobei die Strahlführung 66 für drei Bestrahlungsplätze 67 bis 69 mit dem Einfallswinkel α unter 0° und ein Bestrahlungsplatz 70 mit dem Einfallswinkel α unter 45° von oben ausgelegt ist.

Zusammenfassend betrifft die Erfindung einen Teilchenbeschleuniger für die Strahlentherapie mit Ionenstrahlen, wobei durch die Kombination von Optimierungen aller wichtigen Beschleuniger-Komponenten wie Ionenquellen, Injektor-Linearbeschleuniger, Synchrotronring und Strahlführungen, eine Reduktion der Investitionskosten und der Betriebskosten sowie eine Verbesserung der Zuverlässigkeit im Betrieb erreicht wird. Dazu können einige oder alle aufgeführten Verbesserungen kombiniert werden. Ein derartig verbesserter Teilchenbeschleuniger weist folgende Vorteile auf:
(a) kleine Magnetaperturen bei großer Strahlakzeptanz;
(b) geringe Magnetgewichte;
(c) kleine Pulsleistungen und geringer Energieverbrauch für den Betrieb der Synchrotronmagnete; und
(d) optimierte Parameter für die Auslegung und für den Betrieb des Injektions- und des Extraktionssystems für den Ionenstrahl.

### Bezugszeichenliste

- 1-6: gradlinige Strahlstrecke
- 7-12: gebogene Strahlstrecke
- 13-24: Dipolmagnet
- 13/14: Dipolpaar
- 15/16: Dipolpaar
- 17/18: Dipolpaar
- 19/20: Dipolpaar
- 21/22: Dipolpaar
- 23/24: Dipolpaar
- 25-30: horizontal fokussierender Quadrupol
- 31-36: horizontal defokussierender Quadrupol
- 37-42: Sextupol
- 43: Injektionsmittel
- 44: Strahlbeschleunigungsmittel
- 45: Extraktionsmittel
- 46: Biegemagnet
- 47: Fokussiermagnet
- 48: Defokussiermagnet
- 49: Magnetspulenkonfiguration
- 50: spiegelbildliche Hälfte
- 51: Bumper
- 52: Bumper
- 53: Bumper
- 54: elliptisches Profil
- 55: Pohlschuhkonfiguration
- 56: Quadrant
- 60: Extraktionszweig
- 61: elektrostatisches Extraktionsseptum
- 62: elektromagnetisches Extraktionsseptum
- 63: Dipolmagnet
- 64: Quadrupolmagnet
- 65: Quadrupolmagnet
- 66: Strahlführung
- 67: Bestrahlungsplatz
- 68: Bestrahlungsplatz
- 69: Bestrahlungsplatz
- 70: Bestrahlungsplatz
- 71: Arm im Phasenraum
- 72: Arm im Phasenraum
- 73: Arm im Phasenraum
- 74: zentrale Strahlbahn
- 75: Injektionsweg
- 79: Strahlimpuls
- 80: Ionenquelle
- 81: HeNe-Laser
- 82: CO₂-Laser
- 83: Objektiv
- 84: ionenoptische Linse
- 85: Vorbeschleuniger
- 86: Target
- 87: Plasma
- 88: Oberfläche des Targets
- 90: Linearbeschleuniger
- 91: IH-Sektion
- 92: IH-Sektion
- 93: IH-Sektion
- 94: Quadrupol Triplets
- 95: Quadrupol Triplets
- 96: Quadrupol Triplets
- 100: sechszähliger Synchrotronring
- 150: Ionenstrahl
- 151: Strahlrichtung im Synchrotronring
- 152: eintretender Ionenstrahl in ein elektrostatisches Injektionsseptum
- 153: austretender Ionenstrahl aus einem Injektions- septum
- 154: Strahleintritt
- 155: Strahlaustritt
- 156: Innenelektrode
- 157: Injektionsseptum
- 158: strichpunktierte Linie
- 159: Ablenkplatte
- 160: Phasenraum
- 161: Ellipse
- 170: Phasenraum
- 200: Teilchenbeschleuniger

- α: Bestrahlungswinkel
- aₓ: Magnetapertur in horizontaler Richtung
- a_{y}: Magnetapertur in vertikaler Richtung
- A, A₁: Bahnverlauf bei horizontaler Auslenkung in
- A₂, A₃: x-Richtung
- B: Bahnverlauf bei vertikaler Auslenkung in y-Richtung
- b: Bahnlänge im Synchrotron
- C: Rampe
- D-M: Austrittsrichtungen
- N1 - N35: Umläufe des Ionenstrahls
- r: Bahnradius des injizierten Ionenstrahls
- R: Radius der elektrostatischen Ablenkplatte
- x': Winkelkoordinate
- x: Ortskoordinate

## Patentansprüche

1. Teilchenbeschleuniger für die Strahlentherapie mit Ionenstrahlen, wobei der Teilchenbeschleuniger einen sechszähligen Synchrotronring (100) mit sechs geradlinigen Strahlstrecken (1 bis 6)und sechs gebogenen Strahlstrekken (7 bis 12) aufweist, wobei von den sechs geradlinigen Strahlstrecken (1 bis 6):
- auf einer geradlinigen Strahlstrecke (1) Injektionsmittel (43) für das Einführen eines linearbeschleunigten Ionenstrahls in den Synchrotronring (100) angeordnet sind,
- im Verlauf einer anderen geradlinigen Strahlstrecke (5) mindestens ein Beschleunigungsmittel (44) für den Ionenstrahl vorhanden ist,
- an einer weiteren geradlinigen Strahlstrecke (4) Extraktionsmittel (45) für das Extrahieren des nach mehreren Umläufen hochbeschleunigten Ionenstrahls vorgesehen sind,
- jede gebogene Strahlstrecke (7 bis 12) ein Dipolmagnetpaar (13/14, 15/16, 17/18, 19/20, 21/22, 23/24) aufweist,
- zwischen jedem Dipolmagnetpaar (13/14, 15/16, 17/18, 19/20, 21/22, 23/24) ein horizontal defokussierender Quadrupolmagnet (31 bis 36) angeordnet ist, und
- strahlaufwärts vor jedem Dipolmagnetpaar (13/14, 15/16, 17/18, 19/20, 21/22, 23/24) in den geradlinigen Strahlstrecken (1 bis 6) ein horizontal fokussierender Quadrupolmagnet (25 bis 30) vorgesehen ist.

2. Teilchenbeschleuniger nach Anspruch 1, **dadurch gekennzeichnet, dass** jedes Dipolmagnetpaar (13/14, 15/16, 17/18, 19/20, 21/22, 23/24) eine Magnetspulenkonfiguration (49) einer Kombination von Window-Frame-Magnet-Typ und H-Magnet-Typ aufweist.

3. Teilchenbeschleuniger nach einem der vorhergehenden Ansprüche, **dadurch gekennzeichnet, dass** Bumpermagnete (51, 52, 53) außerhalb der geradlinigen Strahlstrecke (1) für die Injektionsmittel (43) in den anderen der sechs geradlinigen Strahlstrecken (2-6) derart angeordnet sind, dass ein Bumpermagnete (51) strahlabwärts der Injektionsmittel (43) und mindestens ein Bumpermagnet (53) strahlaufwärts der Injektionsmittel (43) angeordnet sind.

4. Teilchenbeschleuniger nach Anspruch 3, **dadurch gekennzeichnet, dass** die Bumpermagnete (51, 52, 53) für eine Injektion des Ionenstrahls (150) innerhalb einer begrenzten Zahl von Umläufen (N1 bis N35) Netz- und Steuergeräte aufweisen, die zur Steuerung eines abnehmenden Erregerstroms eine nichtlineare Rampe (C) mit abgeflachtem Verlauf am Ende der Rampe (C) vorsehen.

5. Teilchenbeschleuniger nach einem der vorhergehenden Ansprüche, **dadurch gekennzeichnet, dass** die Injektionsmittel (43) ein elektrostatisches Injektionsseptum (157) aufweisen, dessen gekrümmte elektrostatische Ablenkplatten (159) einen größeren Krümmungsradius (R) aufweisen als der Bahnradius (r) des vorbeschleunigten injizierten und abgelenkten Ionenstrahls (150).

6. Teilchenbeschleuniger nach einem der vorhergehenden Ansprüche, **dadurch gekennzeichnet, dass** strahlaufwärts vor jeder gebogenen Strahlstrecke (7-12) mit Dipolmagnetpaar (13/14, 15/16, 17/18, 19/20, 21/22, 23/24) und Quadrupolen (25 bis 36) ein Sextupolmagnet (37 bis 42) vorgesehen ist.

7. Teilchenbeschleuniger nach Anspruch 6, **dadurch gekennzeichnet, dass** die Erregerströme der einzelnen Sextupolmagnete (37-42) für eine Resonanzextraktion einstellbar sind und die Sextupolmagnete (37-42) mit einem fixierten elektrostatischen Extraktionsseptum (61) als eines der Extraktionsmittel (45) für die Extraktion des Ionenstrahls (150) in Wirkverbindung stehen.

8. Teilchenbeschleuniger nach einem der vorhergehenden Ansprüche, **dadurch gekennzeichnet, dass** die Extraktionsmittel (45) einen einzigen Extraktionsseptummagneten (62) als elektromagnetisches Extraktionsmittel (45) aufweisen.

9. Teilchenbeschleuniger nach einem der vorhergehenden Ansprüche, **dadurch gekennzeichnet, dass** der Teilchenbeschleuniger (200) strahlaufwärts des sechszähligen Synchrotronrings (100) mindestes eine Ionenquelle (80) und einen Injektor-Linearbeschleuniger (90) als Teilchenbeschleuniger-Komponenten aufweist.

10. Teilchenbeschleuniger nach Anspruch 9, **dadurch gekennzeichnet, dass** der Teilchenbeschleuniger (200) als eine Ionenquelle (80) mindestens eine Laser-Ionenquelle für die Erzeugung von Strahlimpulsen (79) von Kohlenstoff-Ionen aufweist.

11. Teilchenbeschleuniger nach Anspruch 9 oder Anspruch 10, **dadurch gekennzeichnet, dass** der Teilchenbeschleuniger (200) als Injektor-Linearbeschleuniger (90) einen Linearbeschleuniger (90) mit IH-Sektionsmodulen (91, 92, 93) und außerhalb von Vakuumanlagen zwischen den der IH-Sektionsmodule (91, 92, 93) Quadrupol-Linsenmodule (95, 96) aufweist.

12. Teilchenbeschleuniger nach einem der vorhergehenden Ansprüche, **dadurch gekennzeichnet, dass** zwischen Bestrahlungsplätzen (67 bis 70) und sechszähligem Synchrotronring (100) eine Strahlführung (66) angeordnet ist, die eine Kompensation der horizontalen Dispersion direkt nach dem Synchrotronring (100) aufweist, oder die für die Verteilung durch vertikale Ablenkung eine separate vertikale Kompensation für die verschiedenen Bestrahlungsplätze (67 bis 70) vorsieht.

13. Teilchenbeschleuniger nach Anspruch 12, **dadurch gekennzeichnet, dass** die Strahlführung (66) für mehrere Bestrahlungsplätze (67 bis 70) mit unterschiedlichen Einfallswinkel α mit 0° ≤ α ≤ 90° vorgesehen ist, wobei 0° ein horizontaler Einfallwinkel α und 90° ein senkrecht von oben eintreffender Einfallswinkel α ist.

## Claims

1. Particle accelerator for the beam therapy with ion beams, wherein the particle accelerator comprises a six-fold synchroton ring (100) with six straight beam lines (1 to 6) and six bent beam lines (7 to 12), wherein of the six straight beam lines (1 to 6) :
- on a straight beam line (1), injection means (43) for the insertion of a linearly accelerated ion beam into the synchroton ring (100) are arranged,
- in the course of another straight beam line (5), at least one acceleration means (44) for the ion beam exists,
- at another straight beam line (4), extraction means (45) for the extraction of the ion beam being highly accelerated after multiple cycles are provided,
- the bent beam lines (7 to 12) comprise a pair of dipole magnets (13/14, 15/16, 17/18, 19/20, 21/22, 23/24),
- a horizontally defocusing quadrupole magnet (31 to 36) is arranged between each pair of dipole magnets (13/14, 15/16, 17/18, 19/20, 21/22, 23/24), and
- a horizontally focusing quadrupole magnet (25 to 30) is provided upstream in front of each pair of dipole magnets (13/14, 15/16, 17/18, 19/20, 21/22, 23/24) within the straight beam lines (1 to 6).

2. Particle accelerator according to claim 1, **characterized in that** each pair of dipole magnets (13/14, 15/16, 17/18, 19/20, 21/22, 23/24) comprises a magnet-coil-configuration (49) of a combination of window-frame-magnet-type and H-magnet-type.

3. Particle accelerator according to one of the preceding claims, **characterized in that** bumper magnets (51, 52, 53) are arranged outside of the straight beam line (1) for the injection means (43) in the others of the six straight beam lines (2-6) such that a bumper magnet (51) is arranged downstream of the injection means (43) and at least one bumper magnet (53) is arranged upstream of the injection means (43).

4. Particle accelerator according to claim 3, **characterized in that** the bumper magnets (51, 52, 53) comprise power supply units and control units for an injection of the ion beam (150) within a limited number of cycles (N1 to N35), which provide for the control of a decreasing excitation current a non-linear ramp (C) with flattened progression at the end of the ramp (C).

5. Particle accelerator according to one of the preceding claims, **characterized in that** the injection means (43) comprise an electrostatic injection septum (157), whose bent electrostatic deflection plates (159) comprise a larger radius of curvature (R) than the path radius (r) of the preaccelerated injected and deflected ion beam (150).

6. Particle accelerator according to one of the preceding claims, **characterized in that** a sextupole magnet (37-42) is provided upstream in front of each bent beam line (7-12) with a pair of dipole magnets (13/14, 15/16, 17/18, 19/20, 21/22, 23/24) and quadrupoles (25 to 36).

7. Particle accelerator according to claim 6, **characterized in that** the excitation currents of the individual sextupole magnets (37-42) are adjustable for a resonance extraction and the sextupole magnets (37-42) are operatively connected to a fixed electrostatic extraction septum (61) as one of the extraction means (45) for the extraction of the ion beam (150).

8. Particle accelerator according to one of the preceding claims, **characterized in that** the extraction means (45) comprise a single extraction septum magnet (62) as electromagnetic extraction means (45).

9. Particle accelerator according to one of the preceding claims, **characterized in that** the particle accelerator (200) comprises upstream of the six-fold synchroton ring (100) at least one ion source (80) and one injector-linear-accelerator (90) as particle accelerating components.

10. Particle accelerator according to claim 9, **characterized in that** the particle accelerator (200) comprises as an ion source (80) at least one laser ion source for the generation of beam pulses (79) of carbon ions.

11. Particle accelerator according to claim 9 or claim 10, **characterized in that** the particle accelerator (200) comprises a linear accelerator (90) with IH-section modules (91, 92, 93) as injector-linear-accelerator (90) and outside of vacuum facilities between the IH-section modules (91, 92, 93) quadrupole lens modules (95, 96).

12. Particle accelerator according to one of the preceding claims, **characterized in that** a beam guidance (66) is arranged between irradiation sites (67 to 70) and the six-fold synchroton ring (100), which comprises a compensation of the horizontal dispersion directly after the synchroton ring (100) or which provides a separate vertical compensation for the different irradiation sites (67 to 70) for the distribution by vertical deflection.

13. Particle accelerator according to claim 12, **characterized in that** the beam guidance (66) is provided for multiple irradiation sites (67 to 70) with differing angle of incidence α with 0° ≤ α ≤ 90° , wherein 0° is a horizontal angle of incidence α and 90° is an upright angle of incidence α coming from above.

## Revendications

1. Accélérateur de particules pour la radiothérapie avec des faisceaux d'ions, lequel accélérateur de particules comporte un sextuple anneau synchrotron (100) ayant six sections de faisceau rectilignes (1 à 6) et six sections de faisceau curvilignes (7 à 12), sachant que parmi les six sections de faisceau rectilignes (1 à 6) :
- des moyens d'injection (43), destinés à l'introduction d'un faisceau d'ions linéairement accéléré dans l'anneau de synchrotron (100), sont disposés sur une section de faisceau rectiligne (1),
- au moins un moyen d'accélération (44) pour le faisceau d'ions est présent dans le tracé d'une autre section de faisceau rectiligne (5),
- des moyens d'extraction (45), destinés à l'extraction du faisceau d'ions fortement accéléré après plusieurs révolutions, sont prévus sur une autre section de faisceau rectiligne (4),
- chaque section de faisceau curviligne (7 à 12) comporte une paire d'aimants dipôles (13/14, 15/16, 17/18, 19/20, 21/22, 23/24),
- un aimant quadripolaire (31 à 36) défocalisé horizontalement est disposé entre chaque paire d'aimants dipôles (13/14, 15/16, 17/18, 19/20, 21/22, 23/24), et
- il est prévu un aimant quadripolaire (25 à 30) focalisé horizontalement dans les sections de faisceau rectilignes (1 à 6) devant chaque paire d'aimants dipôles (13/14, 15/16, 17/18, 19/20, 21/22, 23/24) dans le sens du faisceau.

2. Accélérateur de particules selon la revendication 1, **caractérisé en ce que** chaque paire d'aimants dipôles (13/14, 15/16, 17/18, 19/20, 21/22, 23/24) présente une configuration de bobine d'aimant (49) d'une combinaison de type d'aimant à ouverture rectangulaire et de type d'aimant H.

3. Accélérateur de particules selon l'une quelconque des revendications précédentes, **caractérisé en ce que** des aimants déviateurs lents (51, 52, 53) sont disposés en dehors de la section de faisceau rectiligne (1) pour les moyens d'injection (43) dans les six autres sections de faisceau rectilignes (2-6) de manière telle qu'un aimant déviateur lent (51) soit disposé derrière les moyens d'injection (43) dans le sens du faisceau et qu'au moins un aimant déviateur lent (53) soit disposé devant les moyens d'injection (43) dans le sens du faisceau.

4. Accélérateur de particules selon la revendication 3, **caractérisé en ce que**, pour une injection des faisceaux d'ions (150) à l'intérieur d'un nombre limité de révolutions (N1 à N35), les aimant déviateurs lents (51, 52, 53) comportent des appareils d'alimentation et de commande qui prévoient, pour commander un courant d'excitation décroissant, une rampe (C) non linéaire avec une partie aplatie à la fin de la rampe (C).

5. Accélérateur de particules selon l'une quelconque des revendications précédentes, **caractérisé en ce que** les moyens d'injection (43) comportent un aimant septum d'injection électrostatique (157) dont les plaques de déflexion électrostatiques cintrées (159) présentent un rayon de cintrage (R) plus grand que le rayon de la trajectoire (r) du faisceau d'ions (150) pré-accéléré, injecté et dévié.

6. Accélérateur de particules selon l'une quelconque des revendications précédentes, **caractérisé en ce qu'**il est prévu un aimant hexapolaire (37 à 42), dans le sens du faisceau, devant chaque section de faisceau curviligne (7-12) ayant une paire d'aimants dipôles (13/14, 15/16, 17/18, 19/20, 21/22, 23/24) et quadripôles (25 à 36).

7. Accélérateur de particules selon la revendication 6, **caractérisé en ce que** les courants d'excitation des différents aimants hexapolaires (37 à 42) peuvent être réglés pour une extraction de résonance et que les aimants hexapolaires (37 à 42) sont en relation active avec un aimant à septum d'extraction électrostatique fixe (61) en tant qu'un des moyens d'extraction (45) pour l'extraction du faisceau d'ions (150).

8. Accélérateur de particules selon l'une quelconque des revendications précédentes, **caractérisé en ce que** les moyens d'extraction (45) comportent un seul aimant à septum d'extraction (62) en tant que moyen d'extraction électromagnétique (45).

9. Accélérateur de particules selon l'une quelconque des revendications précédentes, **caractérisé en ce que** l'accélérateur de particules (200) devant l'anneau de synchrotron sextuple (100) dans le sens du faisceau comporte au moins une source d'ions (80) et un accélérateur linéaire injecteur (90) en tant que composants de l'accélérateur de particules.

10. Accélérateur de particules selon la revendication 9, **caractérisé en ce que** l'accélérateur de particules (200) comporte en tant que source d'ions (80) au moins une source d'ions à laser pour générer des impulsions de faisceau (79) d'ions de carbone.

11. Accélérateur de particules selon la revendication 9 ou 10, **caractérisé en ce que** l'accélérateur de particules (200) comporte en tant qu'accélérateur linéaire injecteur (90) un accélérateur linéaire (90) pourvu de modules de section IH (91, 92, 93) et, en dehors des installations de vide, des modules de lentilles quadripolaires (95, 96) entre les modules de section IH (91, 92, 93).

12. Accélérateur de particules selon l'une quelconque des revendications précédentes, **caractérisé en ce que**, entre les emplacements de rayonnement (67 à 70) et l'anneau de synchrotron sextuple (100) est disposé un système de positionnement de faisceau (66) qui présente une compensation de la dispersion horizontale directement derrière l'anneau de synchrotron (100), ou qui prévoit pour la distribution par déviation verticale une compensation verticale distincte pour les différents emplacements de rayonnement (67 à 70).

13. Accélérateur de particules selon la revendication 12, **caractérisé en ce que** le système de positionnement de faisceau (66) est prévu pour plusieurs emplacements de rayonnement (67 à 70) avec différents angles d'incidence α, avec 0° ≤ α ≤ 90°, 0° étant un angle d'incidence α horizontal et 90° un angle d'incidence α vertical venant du haut.
